# EUROPÄISCHE PATENTSCHRIFT

(11) **EP 0 605 677 B1**
(45) Veröffentlichungstag und Bekanntmachung des Hinweises auf die Patenterteilung: **17.06.1998**
(21) Anmeldenummer: 93912542.3
(22) Anmeldetag: 06.07.1993
(51) Int. Cl.: C03C 17/28, C03C 17/34, C04B 41/46, C07C 69/54

(54) **SILICIUM- ODER SILICIUMDIOXID-SUBSTRAT MIT MODIFIZIERTER OBERFLÄCHE UND VERFAHREN ZU DESSEN HERSTELLUNG**
SILICON OR SILICA SUBSTRATE WITH A MODIFIED SURFACE AND PROCESS FOR PRODUCING THE SAME
SUBSTRAT EN SILICIUM OU EN DIOXYDE DE SILICIUM A SURFACE MODIFIEE ET SON PROCEDE DE PRODUCTION

(30) Priorität: 23.07.1992 CH 2327/92; 02.06.1993 CH 1644/93
(43) Veröffentlichungstag der Anmeldung: 13.07.1994
(73) Patentinhaber: OWENS CORNING, Toledo, Ohio 43659 (US)
(72) Erfinder: GUIDOTTI, Bruno, CH-8807 Freienbach (CH); CASERI, Walter, CH-8049 Zürich (CH); SUTER, Urlrich, W., CH-8050 Zürich (CH); SAUR, Wolfgang, CH-8863 Buttikon (CH)
(74) Vertreter: Rottmann, Maximilian R.
(86) Internationale Anmeldenummer: CH9300173
(87) Internationale Veröffentlichungsnummer: WO9402425

(56) Entgegenhaltungen:
- DE-A- 3 724 364
- US-A- 4 657 815
- Keine weiteren einschlägigen Dokumente gefunden

## Beschreibung

Die Erfindung betrifft ein Silicium- oder Siliciumdioxid-Substrat mit modifizierter Oberfläche und Verfahren zu dessen Herstellung.

Bei der Modifizierung von Oberflächen von Silicium- und Siliciumdioxid-Substraten, insbesondere zum Zusammenhang mit der Entwicklung von Klebstoffen oder Haftvermittlern für derartige Substrate, insbesondere Glas, ging man bisher allgemein von der Vorstellung aus, dass die Silicium- bzw. Siliciumdioxid-Oberflächen mit Silanolgruppen und Wasser bedeckt seien. Demzufolge wurden bisher zur Modifizierung von Silicium- bzw. Siliciumdioxid-Oberflächen mehrheitlich Verbindungen, wie Chlorsilane oder Alkoxysilane,oder bicyclische Amidacetale oder amphihpile Verbindungen eingesetzt, von denen angenommen wurde, dass die mit den Silanolgruppen reagieren und mit diesen kovalente Bindungen eingehen. Dementsprechend wurden diese Verfahren "Silanisierung" genannt.

So ist aus US-A-4 657 815 ein Verfahren zur Modifzierung der Oberfläche eines Füllstoffes, z.B. Glas, bekannt, bei welchem die Oberfläche bei erhöhter Temperatur mit einem bicyclischen Amidacetal behandelt wird. Dadurch soll die Verträglichkeit mit organischen Harzen verbessert werde.

Weiter sind aus DE-A1-37 24 364 Filme aus mindestens einer monomolekularen Schicht auf Substraten, wie Glas, bekannt. Zu deren Herstellugng wird das Substrat mit amphiphilen Verbindungen, wie Hexadekan, Fettsäuren, Trifluoroktadekansäure, Monomethyl-okta-dekandioat, Methyl-stearat, Eicosyl-amin, Dioktadecyl-dimethyl-ammoniumbromid oder einem Cyaninfarbstoff, behandelt. Durch solche Schichten soll die Haftung von Materialien, welche auf das Substrat aufgebracht werden, verbessert werden.

Entgegen der erwähnten Auffassung liegt der vorliegenden Erfindung hingegen die Erkenntnis zugrunde, dass die an der Oberfläche eines solchen Substrates, beispielsweise einer Glasoberfläche, vorhandenen Hydroxylgruppen mindestens teilweise Teil von auf der Siliciumdioxid-Oberfläche molekular verankertem Wasser sind.

Aufgabe der Erfindung ist daher die Schaffung von Silicium- und Siliciumdioxid-Substraten, welche in neuartiger Weise mit organischen Verbindungen modifiziert sind, neuartige Eigenschaften aufweisen und insbesondere als Grundlage für die Verankerung von organischen Polymeren dienen können.

Das erfindungsgemässe Silicium- oder Siliciumdioxid-Substrat mit modifizierter Oberfläche ist nun dadurch gekennzeichnet, dass seine Oberfläche mit einem ungesattigten oder epoxidierten Alkohol oder Silylether besetzt ist.

Eine derart modifizierte Oberfläche weist im Vergleich zu der entsprechenden nicht-modifizierten Oberfläche einen wesentlich vergrösserten Rand- oder Kontaktwinkel von Wasser auf, d.h. die Benetzbarkeit ist für Wasser vermindert, für viele organische Stoffe hingegen erhöht.

Das erfindungsgemässe Verfahren zur Herstellung solcher modifizierter Oberflächen ist dadurch gekennzeichnet, dass man die zu modifizierende Oberfläche mit einem entsprechenden Orthoester behandelt.

Als "Orthoester" bezeichnet man bekanntlich die aliphatischen und aromatischen Ester der in freier Form nicht bekannten entsprechenden Orthocarbonsäuren, somit also Verbindungen vom Typ R¹-C[OR²]₃.

Nach den derzeitigen Erkenntnissen wird angenommen, dass sich bei der Behandlung der Oberfläche des Silicium- oder Siliciumdioxid-Substrates aus den Orthoestern ein Alkohol bildet. Dabei wird das Wasser durch Hydrolyse von der Oberfläche eliminiert, und der entstehende Alkohol oder Silylether nimmt anschliessend den Platz des Wassers ein.

Dies kann schematisch wie folgt dargestellt werden:

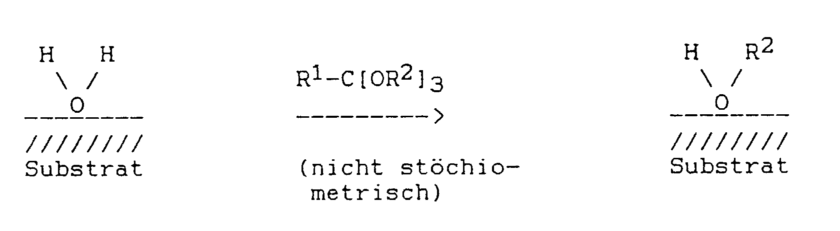

Beispiele der vielfältigen Einsatzgebiete der neuen Technologie sind in der nachstehenden Tabelle 1 zusammengestellt.

Von den dort aufgeführten Anwendungen findet das erfindungsgemässe Verfahren insbesondere Anwendung zur Modifizierung der Oberflächen von Glas, Quarz, Silicium und mit Siliciumdioxid bedecktem Silicium. Bevorzugte Anwendungen betreffen beispielsweise die Modifizierung von pyrogener Kieselsäure. Glasscheiben, Wafern und oxidierten Wafern.

Durch eine solche Behandlung kann beispielsweise die Haftfähigkeit von vielen Polymeren auf dem Substrat auf billige Weise wesentlich verbessert werden. Insbesondere ermöglicht sie auch das direkte Aufpolymerisieren von Klebstoffen und Beschichtungsmassen und von Polymer-Zwischenschichten für die spätere chemische Umsetzung mit Klebstoffen und Beschichtungsmassen.

**Tabelle 1**

| Produktgruppe/Produkte | | Anwendung | Einsätze |
|---|---|---|---|
| 1 | Silikat-Füllstoffe | | |
| 11 | Pyrogene Kieselsäure | Hydrophobierung | Klebstoffe |
| | | Oberflächenbeschichtung | Dichtmassen |
| | | | Kunststoffe |
| | | | Coatings |
| | | | Lacke |
| | | | Farben |
| | | | Formmassen |
| | | | |
| 12 | Gefällte Kieselsäure | Hydrophobierung | Klebstoffe |
| | | Oberflächenbeschichtung | Dichtmassen |
| | | | Kunststoffe |
| | | | Coatings |
| | | | Lacke |
| | | | Farben |
| | | | Formmassen |
| | | | |
| 13 | Silikagele | Hydrophobierung | Katalysatoren |
| | | Oberflächenbeschichtung | Trockungsmittel |
| | | | Chromatographieträger |
| | | | |
| | Quarzmehle | Hydrophobierung | Klebstoffe |
| | | Oberflächenbeschichtung | Dichtmassen |
| | | | Kunststoffe |
| | | | Coatings |
| | | | Lacke |
| | | | Farben |
| | | | Formmassen |
| | | | |
| 15 | Glaspulver | Hydrophobierung | Dentalprodukte |
| | | Oberflächenbeschichtung | Formmassen |
| | | | Composites |
| | | | |
| 16 | Diverse Silikate | Hydrophobierung | Zeolithe |
| | | Oberflächenbeschichtung | |
| | | | |

| 2 | Fasern + Textilien | | |
|---|---|---|---|
| 21 | Glasfasern | Hydrophobierung | Composites |
| | | Schlichten | Glasfaserverstärkte |
| | | Haftvermittler | Kunststoffe |
| | | | Telekomunikation |
| | | | Messtechnik |
| | | | |
| 22 | Glastextilien | Hydrophobierung | Elektrolaminate |
| | | Schlichten | Composites |
| | | Haftvermittler | Glasfaserverstärkte Kunststoffe |
| | | | Industrieschutzanzüge |
| | | | |
| 23 | Glaswolle/Steinwolle | Hydrophobierung | Isoliermaterialien |
| | | | Baustoffe |

| 3 | Flächengläser | | |
|---|---|---|---|
| 31 | Fensterglasverbunde (einschliesslich Isolierglassysteme) | Hydrophobierung | Isolierglasfenster |
| | | Haftvermittler | Sicherheitsverbundglas |
| | | Oberflächenbeschichtung | Autoscheiben |
| | | Korrosionsschutz | |
| 32 | Optische Gläser | Hydrophobierung | Optische Messtechnik |
| | | Haftvermittler | Bildschirme |
| | | Oberflächenbeschichtung | Photographie |
| | | Korrosionsschutz | |
| | | | |
| 33 | Glasmembranen | Hydrophobierung | Chemische Messtechnik |
| | | Haftvermittler | Biotechnologie |
| | | Oberflächenbeschichtung | Chemische Verfahrenstechnik |
| | | Korrosionsschutz | |

| 4 | Spezielle Silikate | | |
|---|---|---|---|
| 41 | Silicium-Wafers für Elektronik und Solartechnik | Hydrophobierung | |
| | | Oberflächenbeschichtung | |
| | | Korrosionsschutz | |
| | | | |
| 42 | Aerogel-Gläser | Hydrophobierung | Technische Isolierschichten |
| | | Oberflächenbeschichtung | Leichtglas für Fahrzeugbau |
| | | Korrosionsschutz | |

"Aerogele" sind neue Entwicklungsmaterialien der Silkatforschung in der Form von transparenten, glasartigen Körpern, z.B. Platten, mit einem spezifischen Gewicht von 0,05 bis 0,1 g/cm³. Sie haben bisher noch keinen industriellen Einsatz gefunden, was auf ihre geringe Hydrolysebestämdigkeit zurückgeführt wird. Durch die erfindungsgemässe Behandlung mit Orthoester kann dieser Mangel behoben werden.

Für eine hydrophobe Beschichtung von Wafers können keine Silane verwendet werden, da diese von ihrer Herstellung her immer Spuren von Chlor enthalten. Dieses ist aber ein starkes Gift für Wafers. Demgegenüber kann die Herstellung von Orthoestern über chlorfreie Verfahren erfolgen.

Die erfindungsgemässe Modifizierung der Oberflächen von Siliciumund Siliciumdioxid-Substraten weist gegenüber der bisherigen Anwendung von Silanen eine Reihe von wesentlichen Vorteilen auf:
- Die Herstellung der Orthoester ist wesentlich billiger als diejenige von Silanen, da sie auf einer organischen Rohstoffbasis, nämlich dem Erdöl, beruhen, und damit energetisch generell günstiger ist als diejenige organischer Siliciumprodukte.
- Sie können nach einfacher zu beherrschenden Herstellungsverfahren, ohne die Anwendung von Chlorchemie, d.h. sicherer und umweltfreundlicher hergestellt werden.
- Zumindest bei einfachen Orthoestern ist deren Anwendung physiologisch unbedenklich.
- Es werden besser überschaubare und analytisch erfassbare Resultate der Behandlung, insbesondere bei Coatings, erhalten.
- Es besteht eine sehr grosse Vielfalt an Synthesemöglichkeiten für anwendungsspezifische Orthoester und damit die Möglichkeit der Herstellung von neuen oder besser angepassten Coatings.
- Da im allgemeinen die C-O-Bindung im Vergleich zur Si-O-Bindung hydrolytisch stabiler ist, werden auch entsprechend resistentere Haftbrücken über die organischen Alkohol-Addukte erhalten.

Der Alkoholteil der genannten Orthoester kann einen Kohlenwasserstoffteil aufweisen, dessen Sequenz gegebenenfalls durch Heteroatome, insbesondere Sauerstoff, unterbrochen ist. Desgleichen kann ein aromatischer Alkoholteil substituiert sein. Insbesondere kann der Alkoholteil epoxidiert sein, d.h. er kann sich von Glycidylalkoholen ableiten.

Benutzt werden Alkoholteile, welche entweder ungesättigt oder epoxidiert sind; man erhält dabei Substratoberflächen, welche durch radikalische Polymerisation bzw. durch Polyaddition mit anderen Substanzen weiter umgesetzt werden können.

Als besonders geeignet haben sich die folgenden Orthoester der allgemeinen Formel

R¹-C[OR²]₃

erwiesen:
1. Triallylorthoformiat
   = Orthoameisensäureallylester
   = Triallyloxymethan
   R¹ = H R² = CH₂-CH=CH₂
2. Tri(ethylacrylat)orthoformiat
   = Orthoameisensäure (ethylacrylat)ester
   = Tri(ethoxyacrylat)methan
   R¹ = H R² = CH₂-CH₂-O-CO-CH=CH₂

Die letztgenannte Verbindung 2 gehört zu einer ersten Gruppe neuer Orthoester, nämlich den Orthoestern der Formel

R¹C[OR²-O-CO-CH=CH₂]₃ (1),

worin R¹ Wasserstoff oder einen organischen Rest und R² (CH₂)ₙ bedeuten, wobei n eine ganze Zahl von 1 bis 18 ist.

Die neuen Orthoester der Formel (1) können hergestellt werden durch Umsetzung eines Säureamids der Formel

R¹-CO-NH₂ (2)

mit Benzamid und dem entsprechenden (2-Hydroxyalkyl)-acrylat. Zweckmässigerweise führt man die Umsetzung als Eintopf-Verfahren durch.

Als besonders geeignet haben sich weiter Orthoester erwiesen, deren Alkoholteile sich von den folgenden Glycidylalkoholen ableiten:
2,3-Epoxy-2-methyl-3-phenyl-1-propanol
   = 2-Methyl-3-phenylglycidol 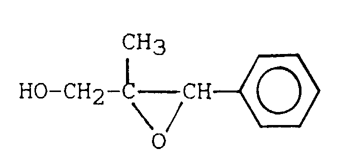 (entsprechend dem Orthoester 3)
2,3-Epoxy-2-methyl-1-propanol 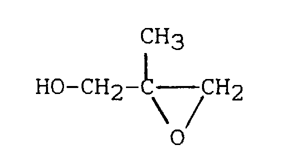 (entsprechend dem Orthoester 4)
2,3-Epoxy-1-propanol 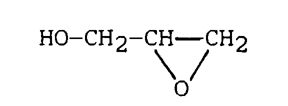 (entsprechend dem Ortoester 5)

Die Orthoester 3 bis 5 gehören zu einer zweiten Gruppe der erfindungsgemässen Orthoester, nämlich den Orthoestern der Formel 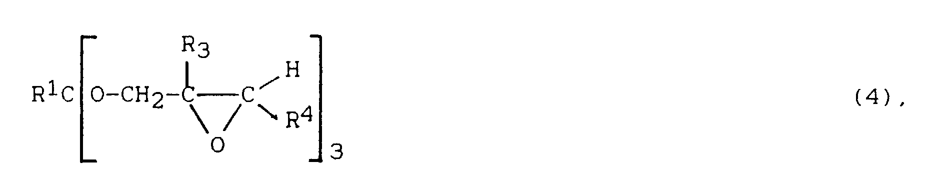 worin
- R¹: Wasserstoff oder einen organischen Rest,
- R³: Wasserstoff oder eine Alkylgruppe mit 1 bis 6 Kohlenstoffatomen; und
- R⁴: Wasserstoff, eine Alkylgruppe oder eine Phenylgruppe bedeuten.

Die Orthoester der Formel (4) werden erfindungsgemäss hergestellt durch Umesterung eines Orthoesters der Formel

R¹C[O-R⁵-CH₃]₃ (8),

worin R⁵ (CH₂)ₘ bedeutet, wobei m eine ganze Zahl von 0 bis 5 ist, mit einem Alkohol der Formel

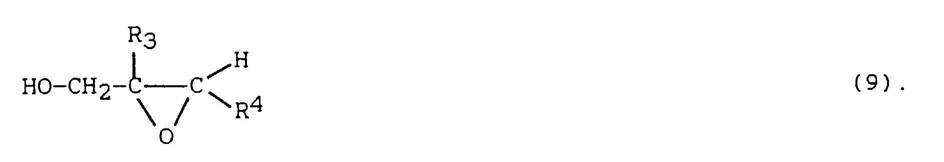

Andere geeignete Orthoester sind in der Veröffentlichung Robert H. DeWolfe, Carboxylic Ortho Acid Derivatives, Academic Press 1970 (Organic Chemistry Series Monographs Nr. 14), beschrieben.

Für die Behandlung mit den genannten Orthoestern wird das Substrat zweckmässigerweise vorgetrockenet, beispielweise bei 150 °C/1 bis 3 mbar. Das Aufbringen selbst erfolgt vorzugsweise bei Raumtemperatur, insbesondere bei Acrylverbindungen, oder am Rückfluss, oder in der Gasphase.

Ist der Alkoholteil des Orthoesters ungesättigt oder epoxidiert, so kann die modifizierte Oberfläche mit anderen reaktiven Substanzen weiter umgesetzt werden.

Auf diese Weise kann auf der modifizierten Substratoberfläche entweder:
- eine Klebstoffschicht oder eine Beschichtungsmasse direkt verankert werden (Substanz-Gruppe 1 hiernach),
oder aber
- eine Polymerschicht mit aktiven Wasserstoffatomen an ihrer Oberfläche erzeugt werden, welche in der Folge mit einem Klebstoff oder einer Beschichtungsmasse, die mit den aktiven Wasserstoffatomen chemische Bindungen einzugehen vermögen, weiter umgesetzt werden (Substanz-Gruppe 2 hiernach).

Für die weitere Umsetzung mit ungesättigten Alkoholteilen geeignete reaktive Substanzen sind beispielsweise:
1 Ungesättigte Verbindungen ohne aktive Wasserstoffatome, insbesondere:
   1.1 Monomere mit mindestens einer olefinischen Doppelbindung:
   1.2 Alkylacrylate und/oder Alkylmethacrylate;
   1.3 Styrol und/oder Acrylnitril;
2 Ungesättigte Verbindungen mit aktiven Wasserstoffatomen, insbesondere:
   2.1 Verbindungen der allgemeinen Formel 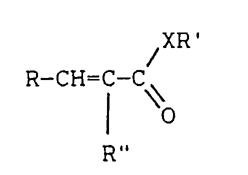 worin bedeuten:
      R und R' unabhängig voneinander ein Wasserstoffatom oder einen Substituenten mit mindestens einem aktiven Wasserstoffatom; und
      R" ein Wasserstoffatom oder einen Substituenten mit mindestens einem aktiven Wasserstoffatom oder einen niedrigen Alkylrest oder CN;
      X ein Sauerstoff- oder Schwefelatom oder den Rest NH; beispielsweise:
         - 2.1.1: Acrylsäure;
         - 2.1.2: 2-Hydroxyethylacrylat;
         - 2.1.3: 4-Hydroxybutylacrylat;
         - 2.1.4: 2,3-Dihydroxypropylacrylat;
         = Glycerylmonoacrylat;
         - 2.1.5: 2,3-Dihydroxypropylmethacrylat
         = Glycerylmonomethacrylat;
         - 2.1.6: Hydroxypropylmethacrylat; oder
         - 2.1.7: Acrylamid;
   2.2 Verbindungen der allgemeinen Formel 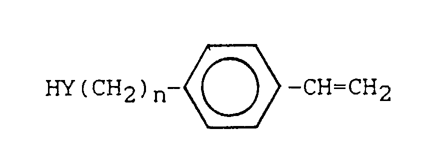 worin bedeuten:
      n eine ganze Zahl von 0 bis 18; und
      Y ein Sauerstoff- oder Schwefelatom oder einen der Reste NH, COO und SO₃; beispielsweise:
         - 2.2.1: 4-Hydroxystyrol; oder
         - 2.2.2: 4-Aminostyrol;
   2.3 ungesättigte Dicarbonsäuren und/oder deren Anhydride; beispielsweise:
      - 2.3.1: Maleinsäure und/oder Maleinsäureanhydrid;
   2.4 Epoxyacrylate und/oder Epoxymethacrylate; beispielsweise
      - 2.4.1: 2,3-Epoxypropylacrylat; oder
      - 2.4.2: 2,3-Epoxypropylmethacrylat.

Die genannten Stoffklassen sind beliebig kombinierbar, d.h. jedes Molekül des behandelten Substrates kann mit Verbindungen jeder der genannten Verbindungsklassen weiter umgesetzt sein.

Die Umsetzung der genannten ungesättigten Verbindungen mit einem auf dem Substrat fixierten ungesättigten Alkohol erfolgt vorzugsweise durch radikalische Polymerisation in Gegenwart von Azoisobuttersäurenitril oder Dibenzoylperoxid als Radikalbildner.

Bei der weiteren Umsetzung der modifizierten Oberflächen mit den genannten reaktiven Substanzen erhält man ein auf der Oberfläche des Siliciumdioxid-Substrates fest verankertes Polymerisat.

Für die weiter Umsetzung von modifizierten Oberflächen, welche mit epoxidierten Alkoholen besetzt sind, eignen sich insbesondere Epoxyverbindungen und Isocyanate.

### Beispiel 1

### Synthese von Triallylorthoformiat = Orthoameisensäureallylester = Triallyloxymethan (Verbindung 1)

Zu einem Gemisch von 45 g (1 mol) Formamid, 174 g (3 mol) Allylalkohol und 200 ml Petrolether wurden unter Kühlen und Rühren innerhalb von 20 min 140,5 g (1 mol) Benzoylchlorid zugetropft. Die Lösung wurde 1 h bei 35 °C gerührt, und das entstandene Ammoniumchlorid und die entstandene Benzoesäure wurden abfiltriert. Das Filtrat wurde unter Rühren und Kühlen in 500 ml Natriumhydroxid-Lösung getropft. Die organische Phase wurde in einem Scheidetrichter abgetrennt, mit 50 ml Wasser gewaschen und über Nacht über CaH₂ getrocknet.
Ausbeute: 123,7 g (61 % d.Th.)

| | | | | |
|---|---|---|---|---|
| ¹H-NMR | s | 5,30 ppm (1 H) | ¹³C-NMR | 111,53 ppm |
| | dxt | 4,12 ppm (6 H) | | 65,13 ppm |
| | dxdxt | 5,93 ppm (3 H) | | 134,05 ppm |
| | dxdxt | 5,18 ppm | | 116,98 ppm |
| | dxdxt | 5,31 ppm (3 H) | | |

### Beispiel 2

### Synthese von Triglycidylorthoformiat (Verbindung 5)

Zu einem Gemisch von 3.15 g (0,297 mol) Trimethylorthoformiat (Verbindung 1 - Sdp.₇₅ = 44 °C) und 106,5 g (1,44 mol) Glycidol (Sdp._{0,006} = 26 °C) wurden 30 mg p-Toluolsulfonsäure-monhydrat, gelöst in 1 ml Methanol, zugegeben.

Das Reaktionsgemisch wurde auf 110 °C erhitzt, so dass die klare Lösung leicht kochte. über ein Vigreux-Kolonne (10 cm) destillierte dabei während rund 1 h ca. 12 ml (0,3 mol) entstandenes Methanol ab. In den folgenden 5 h wurde der Druck mit dem Wasserstrahlvakuum langsam aber kontinuierlich auf 100 mbar gesenkt, so dass die Reaktionslösung immer leicht kochte, die Dampftemperatur aber die Siedetemperatur von Methanol nicht überschritt. Um die Reaktion zu vervollständigen wurden noch weitere 3 h bei 110 °C und 100 mbar gekocht (totale Reaktionsdauer: 9 h). Insgesamt konnten ca. 28 ml (o,7 mol) Methanol abgetrennt werden.

Nachdem überschüssiges Glycidol und Nebenprodukte abdestilliert worden waren, konnten 14,65 g (0,063 mol) Triglycidylorthoformiat in Form einer klaren, öligen Flüssigkeit bei 0,007 Torr und einer Dampftemperatur von 138 °C destilliert werden.
Analyse des Triglycidylorthoformiates:
Ausbeute: 14,65 g (21 % d.Th.)
Sdp._{0,07}: 138 °C
n_{D}²⁰: 1,463

| Mikroanalyse | C | H | O |
|---|---|---|---|
| berechnet | 51,72 % | 6,94 % | 41,34 % |
| gefunden | 51,74 % | 6,80 % | 41,48 % |

FT-IR (gemessen als Flüssigkeitsfilm zwischen 2 Salzplatten)

| | |
|---|---|
| 3060 w, 3002 m | C-H st (Epoxid) |
| 2932 m, 2888 m | C-H st (C-C-H) |
| 1483 w, 1458 w, 1428 w | -CH₂ delta |
| 1256 m | C-O-C st as (Epoxid) |
| 1101 s, 1063 s | C-O-C st as (HC-O-CH₂) |

¹H-NMR (200 MHz, CDCl₃):
delta (Hₐ) = 3,83 ppm
delta (H_{b}) = 3,83 ppm
delta (H_{c}) = 3,48 ppm
delta (H_{d}) = 3,13 ppm
delta (Hₑ) = 2,76 ppm
delta (H_{f}) = 2,58 ppm 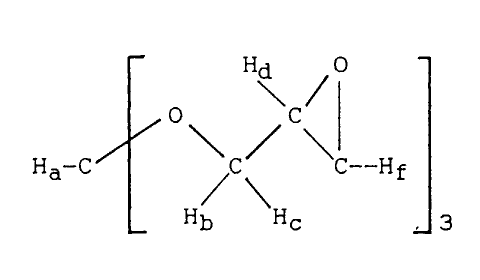
¹³C-NMR (50 MHz, CDCl₃):
delta (Cₐ) = 112 ppm
delta (C_{b}) = 65 ppm
delta (C_{c}) = 50 ppm
delta (C_{d}) = 44 ppm

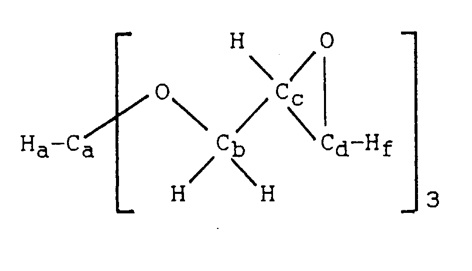

### Bemerkung:

Da es sich beim Kohlenstoffatom C_{c} um ein chirales Zentrum handelt, gibt es vom Triglycidylorthoformiat 2 Enantiomerenpaare, welche im NMR leicht unterschiedliche chemische Verschiebungen zeigen.

### Beispiel 3

### Behandlung von Substraten mit Orthoestern

Glasplatten, Silicium, Siliciumdioxid und Aerosil 200 wurden bei 10-2 mbar getrocknet, über Nacht in 2%ige Orthoesterlösungen in CC14 eingetaucht und über Nacht unter Rückfluss erhitzt. Im Falle von Aerosil wurden die Reaktionsprodukte mittels IR- und ¹³C-Festkörper-NMR-Spektroskopie sowie TGA (Thermogravimetrische Analyse) untersucht. Der Gewichtsanteil an organischem Produkt auf Aerosil 200 betrug typischerweise ca. 3 %.

Die zentralen Orthoester-Kohlenstoffatome waren in allen Fällen im ¹³C-NMR-Spektrum verschwunden; nur die Signale der betreffenden Alkohole waren sichtbar.

Bei Verbindungen der Formel RC[OR']₃ waren auf dem Substrat im Falle R = Aryl, R' = Alkylen im IR-Spektrum nur C-H-Schwingungen von Alkylengruppen zu beobachten. Im Falle R = Alkylen, R' = Aryl waren im IR-Spektrum nur Signale von aromatischen C-H-Streckschwingungen vorhanden.

### Beispiel 4

### Behandlung von Aerosil 200 mit Tri(2,3-epoxy-1-propyl)orthoformiat = Orthoameisensäureglycidylester = Triglycidylorthoformiat

Das Aerosil 200 wurde bei 120 °C/10⁻² mbar während 1 h getrocknet, in eine 10%ige Lösung des genannten Orthoesters in CCl4 eingetaucht und während 3 h bei Raumtemperatur gerührt. Das Reaktionsprodukt wurde mittels IR- und ¹³C-Festkörper-NMR-Spektroskopie sowie TGA (Thermogravimetrische Analyse) untersucht. Der Gewichtsanteil an organischem Produkt auf dem Aerosil 200 betrug typischerweise ca. 3 bis 5 %.

Das zentrale Orthoester-Kohlenstoffatom war in allen Fällen im ¹³C-NMR-Spektrum verschwunden; nur die ¹³C-Signale des betreffenden Epoxyalkohols waren sichtbar.

### Beispiel 5

### Polymerisation

130 mg Dibenzoylperoxid (aus CHCl3/MeOH umkristallisiert) wurden in 100 ml frisch destilliertem Styrol und 20 ml frisch destillierter Methacrylsäure gelöst.

Glasplatten, Silicium, Siliciumdioxid und Aerosil, welche nach der Vorschrift von Beispiel 2 mit Tri(ethylacryl)orthoformiat behandelt worden waren, wurden bei 10⁻² mbar getrocknet und dann 8 h bei 60 °C mit einer Styrol/Methacrylsäure/Dibenzoylperoxid-Lösung umgesetzt. Der Gewichtsanteil an organischem Produkt auf Aerosil betrug 15 %. Dieser Gewichtsanteil veränderte sich auch nach 3tägigem Waschen mit Tetrahydrofuran, Chloroform, Tetrachlorkohlenstoff, Dimethylsulfoxid oder Toluol nicht. Im IR-Spektrum waren Signale von Säuregruppen und Phenylringen identifizierbar.

### Beispiel 6

In der Folge werden die Resultate von Messungen der Rand- oder Kontaktwinkel von Wasser auf verschiedenen Substraten unmittelbar nach der Behandlung mit den angegebenen Orthoestern und/oder nach Einstellen der behandelten Oberflächen mit verschiedenen Lösungsmitteln im Ultraschallbad wiedergegeben.

Als "Si(300ÅOx)" wird eine Silicium-Schicht mit einer Siliciumdioxyd-Oberflächenschicht von 300 Å Dicke bezeichnet.

Die Extraktionsmittel sind abgekürzt wie folgt bezeichnet:

| | |
|---|---|
| CCl₄ | Tetrachlorkohlenstoff = Tetrachlormethan |
| EtOH | Ethanol |
| THF | Tetrahydrofuran = Tetramethylenoxid |
| DMSO | Dimethylsulfoxid |
| H₂O | Wasser |

| 6.4 Triallylorthoformiat = Orthoameisensäureallylester = Triallyloxymethan | | | | | |
|---|---|---|---|---|---|
| Extraktion | | Glas | Quarz | Si(300ÅOx) | Silicium |
| CCl₄ | 15 min | 45° | 50° | 66° | 93° |
| CCl₄ | 15 h | 46° | 47° | 67° | 93° |
| EtOH | 15 min | 48° | 43° | 66° | 93° |
| THF | 15 min | 46° | 45° | 64° | 90° |
| Aceton | 15 min | 46° | 42° | 62° | 90° |
| DMSO | 15 min | 44° | 44° | 63° | 90° |
| H₂O | 15 min | 39° | 30° | 49° | 87° |
| Unbehandelt | | 8° | 14° | 46° | 42...70° |

Dieses Beispiel zeigt, dass die mittels der Behandlung mit Orthoestern auf die Oberflächen aufgebrachten Substanzen nicht nur sehr extraktionsbeständig sind, sondern dass die Rand- oder Kontaktwinkel durch die Extraktion in manchen Fällen sogar noch vergrössert und die Benetzbarkeit somit herabgesetzt wird. Die erklärt sich dadurch, dass die Lösungsmittel adsorbierte polare Verbindungen von der Oberfläche wegwaschen.

### Beispiel 7

| Zugscherprüfung in Anlehnung an DIN 54451 | |
|---|---|
| Klebstoff | BETASEAL^{(R)} HV-3 (eingetragenes Warenzeichen der Gurit-Essex AG, CH-8807 Freienbach) |
| Klebefläche | 25x10 mm |
| Höhe der Kleberaupe | 2 mm |
| Verklebte Materialien | Elektrotauchgrundiertes Blech |
| | 100x25x1 mm; |
| | und |
| | Glas 100x25x5 mm |

- A =: unbehandelt
- B =: mit BETASEAL^{(R)} WIPE VP 04604 (eingetragenes Warenzeichen der Gurit-Essex AG, CH-8807 Freienbach) behandelt
- C =: nach Beispiel 4 behandelt

- Aushärtung:: 7 Tage bei 23 °C/50 % relativer Luftfeuchtigkeit

| | A | B | C |
|---|---|---|---|
| Zugscherfestigkeit [N/mm²] | 1,1 | 6,2 | 6,3 |
| Bruchart | adhäsiv | kohäsiv | kohäsiv |

- Alterung:: 7 Tage bei 70 °C/100 % relativer Feuchtigkeit + 1 Tag bei -20 °C

| | | | |
|---|---|---|---|
| Zugscherfestigkeit [N/mm²] | 0,9 | 3,5 | 5,1 |
| Bruchart | adhäsiv | 80 % kohäsiv | kohäsiv |

## Patentansprüche

1. Silicium- oder Siliciumdioxid-Substrat mit modifizierter Oberfläche, dadurch gekennzeichnet, dass seine Oberfläche mit einem ungesättigten oder epoxidierten Alkohol oder Silylether besetzt ist.

2. Substrat nach Anspruch 1, dadurch gekennzeichnet, dass seine Oberfläche mit einem Alkohol oder Silylether, welcher einen ungesättigten aliphatischen Rest aufweist, besetzt ist.

3. Substrat nach Anspruch 1, dadurch gekennzeichnet, dass seine Oberfläche mit Allylalkohol oder (2-Hydroxyethyl)acrylat oder einem Silylether derselben oder mit einem Glycidylalkohol oder einem Glycidylsilylether besetzt ist.

4. Substrat nach Anspruch 3, dadurch gekennzeichnet, dass seine Oberfläche mit einem ungesättigten Alkohol oder Silylether, der mit einer oder mehreren ungesättigten Verbindungen umgesetzt ist, besetzt ist.

5. Substrat nach Anspruch 4, dadurch gekennzeichnet, dass der Alkohol oder der Silylether mit einer oder mehreren ungesättigten Verbindungen ohne aktive Wasserstoffatome, insbesondere solchen mit mindestens einer olefinischen Doppelbindung oder mit Alkylacrylaten und/oder Alkylmethacrylaten oder mit Styrol und/oder Acrylnitril umgesetzt ist.

6. Substrat nach Anspruch 4, dadurch gekennzeichnet, dass der Alkohol oder der Silylether mit einer oder mehreren ungesättigten Verbindungen mit aktiven Wasserstoffatomen umgesetzt ist.

7. Substrat nach Anspruch 6, dadurch gekennzeichnet, dass der Alkohol oder der Silylether mit einer oder mehreren ungesättigten Verbindungen der allgemeinen Formel 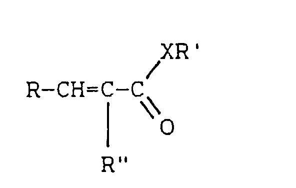 worin
R und R' unabhängig voneinander ein Wasserstoffatom oder einen Substituenten mit mindestens einem aktiven Wasserstoffatom;
R" ein Wasserstoffatom oder einen Substituenten mit mindestens einem aktiven Wasserstoffatom oder einen niedrigen Alkylrest oder CN; und
X ein Sauerstoff- oder Schwefelatom oder den Rest NH
bedeuten, vorzugsweise mit Acrylsäure; 2-Hydroxyethylacrylat; 4-Hydroxybutylacrylat; 2,3-Dihydroxypropylacrylat; 2,3-Dihydroxypropylmethacrylat; und/oder Hydroxypropylmethacrylat, umgesetzt ist.

8. Substrat nach Anspruch 6, dadurch gekennzeichnet, dass der Alkohol oder der Silylether mit Acrylamid umgesetzt ist.

9. Substrat nach Anspruch 6, dadurch gekennzeichnet, dass der Alkohol oder der Silylether mit einer oder mehreren ungesättigten Verbindungen der allgemeinen Formel 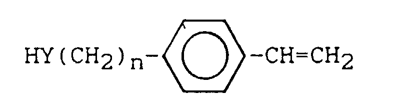 worin
n eine ganze Zahl von 0 bis 18; und
Y ein Sauerstoff- oder Schwefelatom oder einen der Reste NH, COO und SO₃;
bedeuten, umgesetzt ist.

10. Substrat nach Anspruch 9, dadurch gekennzeichnet, dass der Alkohol oder der Silylether mit 4-Hydroxystyrol oder 4-Aminostyrol umgesetzt ist.

11. Substrat nach Anspruch 6, dadurch gekennzeichnet, dass der Alkohol oder der Silylether mit einer oder mehreren ungesättigten Dicarbonsäuren und/oder deren Anhydriden, insbesondere mit Maleinsäure und/oder Maleinsäureanhydrid, umgesetzt ist.

12. Substrat nach Anspruch 6, dadurch gekennzeichnet, dass der Alkohol oder der Silylether mit einem oder mehreren Epoxyacrylaten und/oder Epoxymethacrylaten, insbesondere mit 2,3-Epoxypropylacrylat oder 2,3-Epoxypropylmethacrylat, umgesetzt ist.

13. Substrat nach einem der Ansprüche 1 bis 12, dadurch gekennzeichnet, dass das Substrat ein Flächenglas, insbesondere ein Fensterglasverbund, vorzugsweise ein Isolierglassystem, oder ein optisches Glas oder eine Glasmembran, ist.

14. Substrat nach einem der Ansprüche 1 bis 12, dadurch gekennzeichnet, dass das Substrat ein Silicium-Wafer, insbesondere ein oxidierter Wafer, ist.

15. Substrat nach einem der Ansprüche 1 bis 12, dadurch gekennzeichnet, dass das Substrat ein Aerogel-Glas ist.

16. Verfahren zur Herstellung eines Substrates nach Anspruch 1, dadurch gekennzeichnet, dass man die zu modifizierende Oberfläche mit einem entsprechenden Orthoester behandelt.

17. Verfahren nach Anspruch 16, dadurch gekennzeichnet, dass man die zu modifizierende Oberfläche mit einem Orthoester mit gesättigten Alkoholteil, insbesondere mit einem Alkoholteil, welcher einen gesättigten aliphatischen Rest aufweist, vorzugsweise einem solchen, welcher sich von Methanol, Ethanol einem Propanol oder einem Butanol, oder einem Silylether derselben, oder einem Glycidylalkohol oder Glycidylsilylether, ableitet.

18. Verfahren nach Anspruch 16, dadurch gekennzeichnet, dass man die zu modifizierende Oberfläche mit einem Orthoester mit ungesättigtem Alkoholteil, insbesondere einem solchen, dessen Alkoholteil einen ungesättigten aliphatischen Rest aufweist, vorzugweise einen solchen, welcher sich von Allylalkohol oder (2-Hydroxyethyl)-acrylat ableitet, behandelt.

19. Verfahren nach Anspruch 18, dadurch gekennzeichnet, dass man die modifizierte Oberfläche mit einer oder mehreren ungesättigten Verbindungen umsetzt.

20. Verfahren nach Anspruch 19, dadurch gekennzeichnet, dass man die modifizierte Oberfläche mit einer oder mehreren ungesättigten Verbindungen ohne aktive Wasserstoffatome, oder Alkylacrylaten und/oder Alkylmethacrylaten oder mit Styrol und/oder Acrylnitril umsetzt.

21. Verfahren nach Anspruch 19, dadurch gekennzeichnet, dass man die modifizierte Oberfläche mit einer oder mehreren ungesättigten Verbindungen mit aktiven Wasserstoffatomen umsetzt.

22. Verfahren nach Anspruch 21, dadurch gekennzeichnet, dass man die modifizierte Oberfläche mit einer oder mehreren ungesättigten Verbindungen der allgemeinen Formel 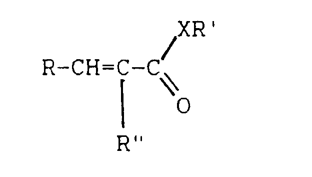 worin
R und R' unabhängig voneinander ein Wasserstoffatom oder einen Substituenten mit mindestens einem aktiven Wasserstoffatom; und
R" ein Wasserstoffatom oder einen Substituenten mit mindestens einem aktiven Wasserstoffatom oder einen niederen Alkylrest oder CN;
X ein Sauerstoff- oder Schwefelatom oder den Rest NH;
bedeuten, insbesondere mit Acrylsäure; 2-Hydroxymethylacrylat; 4-Hydroxybutylacrylat; 2,3-Dihydroxypropylacrylat; 2,3-Dihydroxypropylmethacrylat; und/oder Hydroxypropylmethacrylat, umsetzt.

23. Verfahren nach Anspruch 21, dadurch gekennzeichnet, dass man die modifizierte Oberfläche mit Acrylamid umsetzt.

24. Verfahren nach Anspruch 21, dadurch gekennzeichnet, dass man die modifizierte Oberfläche mit einer oder mehreren ungesättigten Verbindungen der allgemeinen Formel 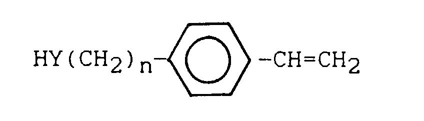 worin
n eine ganze Zahl von 0 bis 18; und
Y ein Sauerstoff- oder Schwefelatom oder einen der Reste NH, COO und SO₃;
bedeuten, umsetzt.

25. Verfahren nach Anspruch 24, dadurch gekennzeichnet, dass man die modifizierte Oberfläche mit 4-Hydroxystyrol oder 4-Aminostyrol umsetzt.

26. Verfahren nach Anspruch 21, dadurch gekennzeichnet, dass man die modifizierte Oberfläche mit einer oder mehreren ungesättigten Dicarbonsäuren und/oder deren Anhydriden, insbesondere mit Maleinsäure und/oder Maleinsäureanhydrid, umsetzt.

27. Verfahren nach Anspruch 21, dadurch gekennzeichnet, dass man die modifizierte Oberfläche mit einem oder mehreren Epoxyacrylaten und/oder Epoxymethacrylaten, insbesondere mit 2,3Epoxypropylacrylat oder 2,3-Epoxypropylmethacrylat, umsetzt.

## Claims

1. Silicon or silica substrate with a modified surface, characterized in that its surface is occupied by an unsaturated or epoxidized alcohol or silyl ether.

2. Substrate according to Claim 1, characterized in that its surface is occupied by an alcohol or silyl ether which has an unsaturated aliphatic radical.

3. Substrate according to Claim 1, characterized in that its surface is occupied by allyl alcohol or 2-hydroxyethyl acrylate or a silyl ether thereof or by a glycidyl alcohol or a glycidyl silyl ether.

4. Substrate according to Claim 3, characterized in that its surface is occupied by an unsaturated alcohol or silyl ether that has been reacted with one or more unsaturated compounds.

5. Substrate according to Claim 4, characterized in that the alcohol or the silyl ether has been reacted with one or more unsaturated compounds devoid of active hydrogen atoms, especially those having at least one olefinic double bond, or with alkyl acrylates and/or alkyl methacrylates or with styrene and/or acrylonitrile.

6. Substrate according to Claim 4, characterized in that the alcohol or the silyl ether has been reacted with one or more unsaturated compounds having active hydrogen atoms.

7. Substrate according to Claim 6, characterized in that the alcohol or the silyl ether has been reacted with one or more unsaturated compounds of the general formula 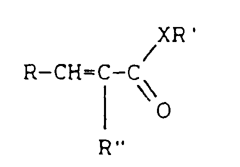 in which
R and R' independently of one another are a hydrogen atom or a substituent having at least one active hydrogen atom;
R" is a hydrogen atom or a substituent having at least one active hydrogen atom or is a lower alkyl radical or CN; and
X is an oxygen or sulphur atom or the radical NH,
preferably with acrylic acid; 2-hydroxyethyl acrylate; 4-hydroxybutyl acrylate; 2,3-dihydroxypropyl acrylate; 2,3-dihydroxypropyl methacrylate; and/or hydroxypropyl methacrylate.

8. Substrate according to Claim 6, characterized in that the alcohol or the silyl ether has been reacted with acrylamide.

9. Substrate according to Claim 6, characterized in that the alcohol or the silyl ether has been reacted with one or more unsaturated compounds of the general formula 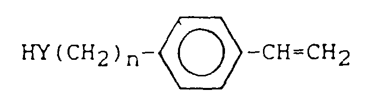 in which
n is an integer from 0 to 18; and
Y is an oxygen or sulphur atom or one of the radicals NH, COO and SO₃.

10. Substrate according to Claim 9, characterized in that the alcohol or the silyl ether has been reacted with 4-hydroxystyrene or 4-aminostyrene.

11. Substrate according to Claim 6, characterized in that the alcohol or the silyl ether has been reacted with one or more unsaturated dicarboxylic acids and/or their anhydrides, in particular with maleic acid and/or maleic anhydride.

12. Substrate according to Claim 6, characterized in that the alcohol or the silyl ether has been reacted with one or more epoxy acrylates and/or epoxy methacrylates, in particular with 2,3-epoxypropyl acrylate or 2,3-epoxypropyl methacrylate.

13. Substrate according to one of Claims 1 to 12, characterized in that the substrate is a glass sheet, especially a window glass laminate, preferably an insulating glass system, or an optical glass or a glass membrane.

14. Substrate according to one of Claims 1 to 12, characterized in that the substrate is a silicon wafer, especially an oxidized wafer.

15. Substrate according to one of Claims 1 to 12, characterized in that the substrate is an aerogel glass.

16. Process for preparing a substrate according to Claim 1, characterized in that the surface to be modified is treated with a corresponding ortho ester.

17. Process according to Claim 16, characterized in that the surface to be modified is treated with an ortho ester having a saturated alcohol moiety, in particular having an alcohol moiety that has a saturated aliphatic radical, preferably one which is derived from methanol, ethanol, a propanol or a butanol, or a silyl ether thereof, or from a glycidyl alcohol or glycidyl silyl ether.

18. Process according to Claim 16, characterized in that the surface to be modified is treated with an ortho ester having an unsaturated alcohol moiety, in particular one whose alcohol moiety has an unsaturated aliphatic radical, preferably one which is derived from allyl alcohol or 2-hydroxyethyl acrylate.

19. Process according to Claim 18, characterized in that the modified surface is reacted with one or more unsaturated compounds.

20. Process according to Claim 19, characterized in that the modified surface is reacted with one or more unsaturated compounds devoid of active hydrogen atoms, or alkyl acrylates and/or alkyl methacrylates or with styrene and/or acrylonitrile.

21. Process according to Claim 19, characterized in that the modified surface is reacted with one or more unsaturated compounds having active hydrogen atoms.

22. Process according to Claim 21, characterized in that the modified surface is reacted with one or more unsaturated compounds of the general formula 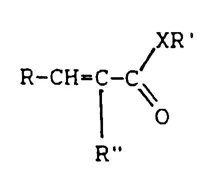 in which
R and R' independently of one another are a hydrogen atom or a substituent having at least one active hydrogen atom;
R" is a hydrogen atom or a substituent having at least one active hydrogen atom or is a lower alkyl radical or CN; and
X is an oxygen or sulphur atom or the radical NH,
in particular with acrylic acid; 2-hydroxyethyl acrylate; 4-hydroxybutyl acrylate; 2,3-dihydroxypropyl acrylate; 2,3-dihydroxypropyl methacrylate; and/or hydroxypropyl methacrylate.

23. Process according to Claim 21, characterized in that the modified surface is reacted with acrylamide.

24. Process according to Claim 21, characterized in that the modified surface is reacted with one or more unsaturated compounds of the general formula 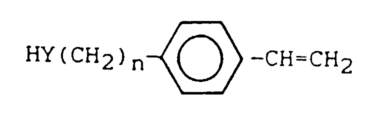 in which
n is an inteqer from 0 to 18; and
Y is an oxygen or sulphur atom or is one of the radicals NH, COO and SO₃.

25. Process according to Claim 24, characterized in that the modified surface is reacted with 4-hydroxystyrene or 4-aminostyrene.

26. Process according to Claim 21, characterized in that the modified surface is reacted with one or more unsaturated dicarboxylic acids and/or their anhydrides, in particular with maleic acid and/or maleic anhydride.

27. Process according to Claim 21, characterized in that the modified surface is reacted with one or more epoxy acrylates and/or epoxy methacrylates, in particular with 2,3-epoxypropyl acrylate or 2,3-epoxypropyl methacrylate.

## Revendications

1. Substrat en silicium ou en dioxyde de silicium à surface modifiée, caractérisé en ce que sa surface est occupée par un alcool insaturé ou époxydé ou bien par un éther silylique.

2. Substrat selon la revendication 1, caractérisé en ce que sa surface est occupée par un alcool ou par un éther silylique présentant un reste aliphatique insaturé.

3. Substrat selon la revendication 1, caractérisé en ce que sa surface est occupée par de l'alcool allylique ou de l'acrylate de 2-hydroxyéthyle ou un éther silylique de ceux-ci ou bien un alcool glycidylique ou un éther glycidylsilylique.

4. Substrat selon la revendication 3, caractérisé en ce que sa surface est occupée par un alcool insaturé ou un éther silylique ayant réagi avec un ou plusieurs composés insaturés.

5. Substrat selon la revendication 4, caractérisé en ce que l'alcool ou l'éther silylique a réagi avec un ou plusieurs composés insaturés dépourvus d'atomes d'hydrogène actifs, notamment ceux possédant au moins une double liaison oléfinique, ou avec des acrylates et/ou des méthacrylates d'alkyles ou bien avec du styrène et/ou de l'acrylonitrile.

6. Substrat selon la revendication 4, caractérisé en ce que l'alcool ou l'éther silylique a réagi avec un ou plusieurs composés insaturés possédant des atomes d'hydrogène actifs.

7. Substrat selon la revendication 6, caractérisé en ce que l'alcool ou l'éther silylique a réagi avec un ou plusieurs composés insaturés de la formule générale 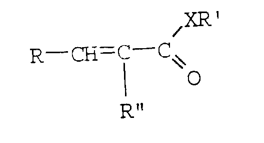 dans laquelle :
R et R' sont indépendamment l'un de l'autre un atome d'hydrogène ou un substituant possédant au moins un atome d'hydrogène actif ;
R" est un atome d'hydrogène ou un substituant possédant au moins un atome d'hydrogène actif ou bien un reste alkyle faible ou bien CN ; et
X est un atome d'oxygène ou de soufre ou bien le reste NH,
de préférence avec de l'acide acrylique ; de l'acrylate de 2-hydroxyéthyle ; de l'acrylate de 2hydroxybutyle ; de l'acrylate de 2,3-dihydroxypropyle ; du méthacrylate de 2,3-dihydroxypropyle ; et/ou du méthacrylate d'hydroxypropyle.

8. Substrat selon la revendication 6, caractérisé en ce que l'alcool ou l'éther silylique a réagi avec de l'acrylamide.

9. Substrat selon la revendication 6, caractérisé en ce que l'alcool ou l'éther silylique a réagi avec un ou plusieurs composés insaturés de la formule générale 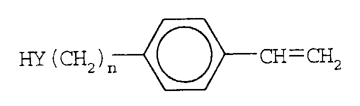 dans laquelle :
n est un nombre entier compris entre 0 et 18 ; et
Y est un atome d'oxygène ou de soufre ou bien l'un des restes NH, COO et SO₃.

10. Substrat selon la revendication 9, caractérisé en ce que l'alcool ou l'éther silylique a réagi avec du 4-hydroxystyrène ou du 4-aminostyrène.

11. Substrat selon la revendication 6, caractérisé en ce que l'alcool ou l'éther silylique a réagi avec un ou plusieurs acides dicarboxyliques et/ou leurs anhydrides, notamment avec l'acide maléique et/ou avec l'anhydride maléique.

12. Substrat selon la revendication 6, caractérisé en ce que l'alcool ou l'éther silylique a réagi avec un ou plusieurs époxyacrylates et/ou époxyméthacrylates, notamment avec de l'acrylate de 2,3-époxypropyle ou du méthacrylate de 2,3-époxypropyle.

13. Substrat selon l'une quelconque des revendications 1 à 12, caractérisé en ce que le substrat est un verre plat, notamment un vitrage composite, de préférence un système de vitrage isolant, ou bien un verre optique ou une membrane en verre.

14. Substrat selon l'une quelconque des revendications 1 à 12, caractérisé en ce que le substrat est une tranche de silicium, notamment une tranche oxydée.

15. Substrat selon l'une quelconque des revendications 1 à 12, caractérisé en ce que le substrat est un verre d'aérogel.

16. Procédé de fabrication d'un substrat selon la revendication 1, caractérisé en ce que l'on traite la surface à modifier avec un orthoester correspondant.

17. Procédé selon la revendication 16, caractérisé en ce que l'on traite la surface à modifier avec un orthoester comportant une partie alcoolique saturée, notamment une partie alcoolique présentant un reste aliphatique saturé, de préférence un reste dérivé du méthanol, de l'éthanol, d'un propanol ou d'un butanol ou bien d'un éther silylique de ceux-ci, ou bien d'un alcool glycidylique ou d'un éther glycidylsilylique.

18. Procédé selon la revendication 16, caractérisé en ce que l'on traite la surface à modifier avec un orthoester comportant une partie alcoolique insaturée, notamment un orthoester dont la partie alcoolique présente un reste aliphatique insaturé, de préférence un reste dérivé de l'alcool allylique ou de l'acrylate de 2-hydroxyéthyle.

19. Procédé selon la revendication 16, caractérisé en ce que l'on fait réagir la surface modifiée avec un ou plusieurs composés insaturés.

20. Procédé selon la revendication 19, caractérisé en ce que l'on fait réagir la surface modifiée avec un ou plusieurs composés insaturés dépourvus d'atomes d'hydrogène actifs ou avec des acrylates et/ou méthacrylates d'alkyles ou avec du styrène et/ou de l'acrylonitrile.

21. Procédé selon la revendication 19, caractérisé en ce que l'on fait réagir la surface modifiée avec un ou plusieurs composés insaturés possédant des atomes d'hydrogène actifs.

22. Procédé selon la revendication 21, caractérisé en ce que l'on fait réagir la surface modifiée avec un ou plusieurs composés insaturés de la formule générale 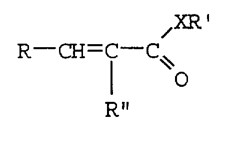 dans laquelle :
R et R' sont indépendamment l'un de l'autre un atome d'hydrogène ou un substituant possédant au moins un atome d'hydrogène actif ;
R" est un atome d'hydrogène ou un substituant possédant au moins un atome d'hydrogène actif ou bien un reste alkyle faible ou bien CN ; et
X est un atome d'oxygène ou de soufre ou bien le reste NH,
de préférence avec de l'acide acrylique ; de l'acrylate de 2-hydroxyéthyle ; de l'acrylate de 2-hydroxybutyle ; de l'acrylate de 2,3-dihydroxypropyle ; du méthacrylate de 2,3-dihydroxypropyle ; et/ou du méthacrylate d'hydroxypropyle.

23. Procédé selon la revendication 21, caractérisé en ce que l'on fait réagir la surface modifiée avec de l'acrylamide.

24. Procédé selon la revendication 21, caractérisé en ce que l'on fait réagir la surface modifiée avec un ou plusieurs composés insaturés de la formule générale 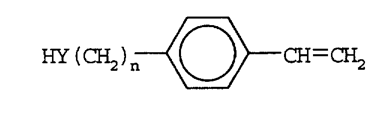 dans laquelle :
n est un nombre entier compris entre 0 et 18 ; et
Y est un atome d'oxygène ou de soufre ou bien l'un des restes NH, COO et SO₃.

25. Procédé selon la revendication 24, caractérisé en ce que l'on fait réagir la surface modifiée avec du 4-hydroxystyrène ou du 4-aminostyrène.

26. Procédé selon la revendication 21, caractérisé en ce que l'on fait réagir la surface modifiée avec un ou plusieurs acides dicarboxyliques et/ou leurs anhydrides, notamment avec l'acide maléique et/ou avec l'anhydride maléique.

27. Procédé selon la revendication 21, caractérisé en ce que l'on fait réagir la surface modifiée avec un ou plusieurs époxyacrylates et/ou époxyméthacrylates, notamment avec de l'acrylate de 2,3-époxypropyle ou du méthacrylate de 2,3-époxy-propyle.
